# EUROPEAN PATENT APPLICATION

(11) **EP 2 402 661 A1**
(43) Date of publication of application: **04.01.2012**
(21) Application number: 10006844.4
(22) Date of filing: 02.07.2010
(51) Int. Cl.: F24C 15/20, F24C 15/32, A47L 15/42, A61L 9/22, F25D 17/04

(54) **Household appliance and method for disinfecting a functional subunit of a household appliance and/or reducing odours**

(71) Applicant: Electrolux Home Products Corporation N.V., 1130 Brussels (BE)
(72) Inventor: Reinhard-Herrscher, Fabienne, 74582 Gerabronn (DE); Kaiser, Kersten, 91541 Rothenburg (DE)
(74) Representative: Baumgartl, Gerhard Willi

(57) **Abstract**

The present application in particular is directed to a household appliance adapted for treating objects in a primary treatment process. For providing easy disinfection of process media and functional subunits of the household appliance, it is proposed that the household appliance comprises at least one plasma generator adapted to impinge in a secondary treatment process a disinfecting and/or odour reducing plasma at at least one functional subunit of the appliance.

## Description

The present application is directed to a household appliance and to a method of disinfecting a household appliance and/or reducing odours with such an appliance.

Household appliances, such as baking ovens, have treatment chambers in which air or steam is present, exchanged or circulated during operation.

In many cases, air or steam exhausted from the treatment chamber to the environment lead to unpleasant odours. It is highly desirable to reduce such odours. In addition, due increasing health consciousness, it is desirable that air or steam used or present or feed to the treatment chamber is at least partially disinfected in order to reduce microorganisms, viruses and fungi. Further it may be desired to disinfect functional subunits of the household appliance from time to time, in particular if foodstuff is processed or treated.

It is therefore an object of the invention to provide a household appliance which allows for disinfection of process media and functional subunits of the household appliance in a comfortable way. Further, a comfortable method of disinfecting process media and functional subunits of a household appliance shall be provided.

This object is achieved by independent claims 1 and 8. Embodiments of the invention result from dependent claims.

According to claim 1, a household appliance is provided which is adapted for treating objects in a primary treatment process.

Such a household appliance may for example be a baking oven, a microwave oven, a refrigerator, a freezer, dishwasher or a tumble dryer. The primary treatment process of a household appliance is understood to be the treatment process conducted by the household appliance in ordinary operation and ordinary use thereof. The ordinary operation and use of the household appliances mentioned beforehand is for example cooking, baking, steaming, cooling, freezing or drying.

The household appliance comprises at least one plasma generator which is adapted to impinge in a secondary treatment process a disinfecting plasma at at least one functional subunit of the appliance. The plasma can be any type of plasma, in particular a so-called cold plasma. The plasma generator can be any known type of plasma generator and may comprise electrodes or electrode grids between which a high voltage is applied for plasma generation.

The term secondary treatment process shall be understood to be different from the primary treatment process, i. e. both processes can be clearly distinguished from each other. However, this shall not exclude to jointly and simultaneously carry out the primary and secondary treatment process. In particular, this shall not mean that both processes always have to be carried out separately and independently from each other.
Providing the plasma generator has the advantage, that respective functional subunits can be easily disinfected by the disinfecting plasma. Impinging the disinfecting plasma on functional subunits, such as ventilation systems, fans, treatment chambers and the like, makes it also possible to disinfect process media, such as exhaust gases, air fed to the household appliance, or air or steam present or circulated in a treatment chamber of the household appliance, for example. With regard to exhaust gases, unwanted odours can be prevented, whereas with regard to air supply and circulation, the number and growth of microorganisms, viruses, fungi and the like fed to or present in respective functional subunits, such as ducting, treatment chambers etc., can be greatly reduced.

As already mentioned the primary process can involve heating foodstuff, in particular cooking or baking as well as steaming, cooling foodstuff and drying clothing or dishes. Respective household appliances are baking ovens, microwave ovens, freezers, refrigerators, tumble dryers, dishwashers and the like. It is even conceivable that the plasma generator is part of a vacuum cleaner with the disinfecting plasma being impinged at exhaust air ducting so as to reduce unwanted odours. In this case the primary treatment process would be a hoovering action. Further household appliances are conceivable.

Within the scope of the present invention, the functional subunit can be a primary process treatment chamber, in particular oven muffle, a primary process heating element, a primary process cooling element, a primary process steam generator, as well as primary process treatment media related storage tanks, ventilation systems, ducting systems, in particular exhaust ducting systems and inlet ducting systems. Such functional subunits in particular relate to household appliances mentioned so far. By impinging the disinfecting plasma at such functional units, unwanted odours and/or the number and growth of microorganisms and the like can be greatly reduced in a comparatively comfortable manner.

In a preferred embodiment, the plasma generator is adapted to generate a plasma in a primary process treatment medium, preferably of gaseous type, preferably air or steam. In this case there is no need to provide extra plasma mediums and respective substances for generating the plasma. Hence, construction and operation of the plasma generator can be greatly simplified. However, it is also possible, that additional plasma mediums and related substances, preferably of gaseous type, are used. Such mediums and substances may be selected and adapted to improve the reactivity and disinfecting efficiency of the plasma. The primary process treatment medium can be one that is present, exhausted from and/or supplied to a primary process treatment chamber of the household appliance.

In an embodiment, the plasma generator comprises at least one plasma generating unit. Such a plasma generating unit may be of conventional type and construction. In particular the plasma generating unit may comprise a pair of electrodes, in particular grid electrodes, adapted to generate a disinfecting and/or odour reducing plasma upon applying high voltages between the electrodes.

The at least one plasma generating unit can be arranged within at least one of the at least one functional subunit, or it can be connected thereto.

In the embodiment, in which the plasma generating unit is connected to the functional subunit, the plasma can be generated in a separate chamber or cavity and impinged at the functional subunit via a ducting system or similar. The ducting system may be designed such that the disinfecting plasma can be impinged at either a respective single one or at several functional subunits. In particular, it is possible that such a ducting system is designed such that the type and number of functional subunits, the disinfecting plasma is impinged at, can be freely selected. Such a selection may be performed by a user, or automatically by household appliance controls, preferably in dependence of operational programs respectively executed by the household appliance.

In a further embodiment, the household appliance comprises a ventilation unit adapted to ventilate the gaseous type treatment medium through at least one of the at least one plasma generating unit. The ventilation unit may be adapted such that the disinfecting and/or odours reducing plasma optimally impinges at respective functional subunits. If for example, the plasma generator is arranged within the treatment chamber, an oven muffle for example, and the ventilation unit is further adapted to circulate air within the treatment chamber, the disinfecting plasma can be optimally impinged at the treatment chamber. This in turn means, that the number of microorganisms, viruses and fungi present in the treatment chamber, on treatment chamber walls or other elements, such as food items placed therein, can be greatly reduced. The same applies to similar household appliances.

In a yet further embodiment, at least one of the at least one plasma generator is integrated into the appliance.

However, in another embodiment it is possible that at least one of the at least one plasma generator is designed as a separate unit releasably connected or connectable to the at least one functional subunit.

The terms connect or connection in particular shall mean any type of connection allowing the plasma generator to impinge disinfecting plasma at one or several functional subunits.

It is further possible that the at least one plasma generator is releasably mounted to the household appliance, and that upon mounting a connection between the plasma generator or a respective plasma generating unit and the functional subunit is automatically established. In the latter cases, the plasma generator may be designed such that it can be used with or connected to different household appliances, in particular different types of household appliances. In this case, the plasma generator and household appliance may have corresponding connector interfaces designed such that the plasma generator can be easily and quickly connected and disconnected.

Independent claim 8 is directed to a method of disinfecting at least a functional subunit of a household appliance, which household appliance is adapted for treating objects in a primary treatment process. According to the proposed method, in a secondary treatment process a disinfecting plasma generated by at least one plasma generator is impinged at at least one functional subunit of the household appliance.

In an embodiment of the method, the functional subunit being selected from the group: primary process treatment chamber, in particular oven muffle, primary process heating element, primary process cooling element, primary process steam generator, ventilation systems, ducting systems, in particular exhaust ducting systems and inlet ducting systems as well as primary process treatment media related storage tanks.

In a further embodiment of the method, the plasma generator generates a plasma in a primary process treatment medium, preferably of gaseous type, preferably air or steam.

In a yet further embodiment of the method, the gaseous type treatment medium is ventilated through a plasma generating unit of the plasma generator by means of a ventilation unit.

As to advantages and advantageous effects of the method and embodiments thereof, reference is made to the description further above.

Embodiments of the invention will now be described in connection with the annexed figures, in which
- Fig. 1: shows a schematic cross sectional side view of a first embodiment of a baking oven; and
- Fig. 2: shows a schematic cross sectional side view of a second embodiment of a baking oven.

The following description of embodiments of a baking oven shall not be construed as limiting the scope of the invention. In this respect a baking oven is just one representative of a household appliance within the scope of the invention. Further, any features jointly shown in connection with the embodiments can be implemented alone or in any other combination as discussed further above. Note, that the embodiments are described only as far as is necessary for understanding the invention.

If not otherwise stated like elements are denoted by like reference signs throughout the figures.

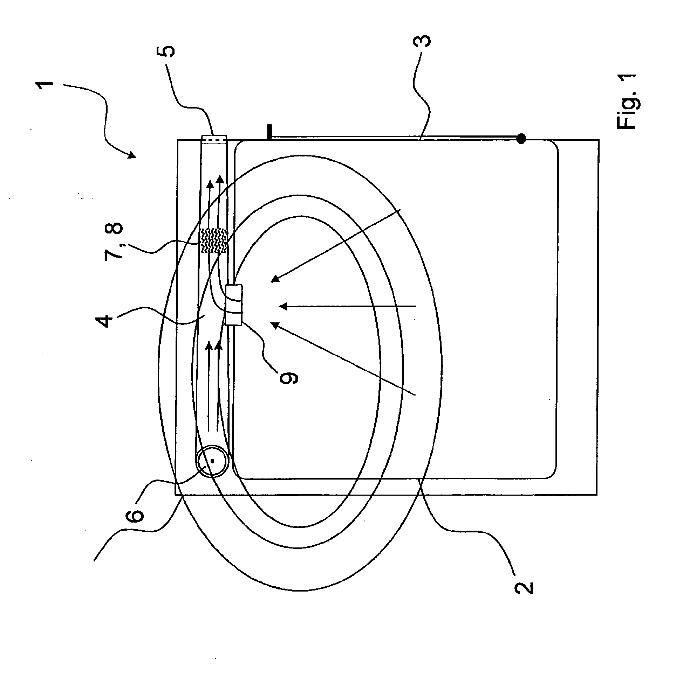

Fig. 1 shows a schematic cross sectional side view of a first embodiment of a baking oven 1. The baking oven 1 comprises an oven muffle 2 with a front opening which is closed via a muffle door 3 in the present case.

The baking oven 1 further comprises an exhaust ducting 4. The exhaust ducting 4 is connected to the oven muffle 2 in an upper section thereof. The exhaust ducting 4 opens out into a ventilation opening 5 provided in the front aperture of the baking oven 1 and being covered by a ventilation grid. Further, a blower 6 or fan for generating an exhaust air stream is connected to the exhaust ducting 4 on a side averted from the ventilation opening 5. The exhaust air stream is indicated by arrows and is such that air inside the oven muffle 2 is sucked from the oven muffle 2 into the exhaust ducting 4 and finally exhausted via the ventilation opening 5.

The baking oven 1 further comprises a plasma generator 7 which presently is arranged within the exhaust ducting 4. The plasma generator 7 comprises a plasma generating unit 8 which is arranged in the exhaust ducting 4 downstream the blower 6 and also downstream the aperture 9 which connects the exhaust ducting 4 and the oven muffle 2.

The plasma generator 7, or plasma generating unit 8 generates a disinfecting and/or odour reducing plasma and impinges the disinfecting plasma at the exhaust ducting 4, and also at the ventilation opening 5 and ventilation grid. In this case, the effect of the disinfecting plasma is twofold. First, the exhaust air stream is blown through the plasma and is thus disinfected in turn reducing unwanted odours blown out from the baking oven 1 to the environment. Second, at least a part of the exhaust ducting 4, more precisely the part of the exhaust ducting 4 downstream the plasma generating unit 8, is disinfected suppressing growth of microorganisms, viruses, fungi and the like.

As can be seen, the baking oven 1 provides a comfortable way for disinfection of process media and functional subunits, in the present case a part of the exhaust ducting 4, of the baking oven 1.

Similar to the embodiment shown and described in connection with Fig. 1, there may be provided a plasma generator in an inlet air ducting (not shown) of the baking oven 1.

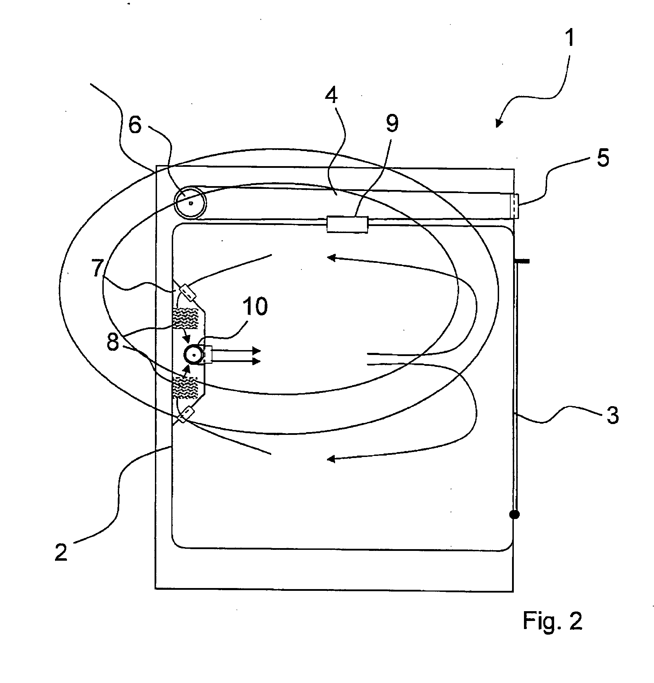

Fig. 2 shows a schematic cross sectional side view of a second embodiment of a baking oven 1. The baking oven 1 of Fig. 2 differs from that of Fig. 1 in that the plasma generator 7 is arranged within the oven muffle 2 and comprises two plasma generating units 8. Conceivably this plasma generator 7 can also comprise just one plasma generating unit 8 that might be round shaped or of any other suitable shape.

The baking oven 1 comprises a separate fan or ventilation unit 10 which is arranged and adapted to guide air within the oven muffle 2 through the plasma generating units 8. In the present case, the ventilation unit 10 sucks in air from the oven muffle 2 through openings provided on opposite sides of a casing of the plasma generator 7. The openings and plasma generating units 8 are arranged such that the air is guided through the plasma generating units 8. Air having passed the plasma generating units 8 is then blown towards the center of the oven muffle 2 by the ventilation unit 10. As is indicated in Fig. 2 by arrows, two circulating air streams of opposite flow directions are generated, covering a relatively large volume of the oven muffle 2.

In the present embodiment, the plasma generator 7, i. e. the plasma generating units 8, generate a disinfecting plasma which is impinged at the oven muffle 2. The effect of the disinfecting plasma with the second embodiment is twofold too. The disinfecting plasma develops a disinfecting effect for air inside the oven muffle 2. Disinfected air circulating in the oven muffle 2 in turn develops a disinfecting effect for the muffle walls of the oven muffle 2 and for foodstuff inserted into the oven muffle 2.

The disinfecting effect leads to reduced growth and reduced number of microorganisms, viruses and fungi. Also, air inside the oven muffle circulated and disinfected as described beforehand hardly causes any unwanted odours if exhausted via the exhaust ducting 4.

The plasma generator 7 can be operated during baking foodstuff, in particular to reduce odours. However, it is also possible that the plasma generator 7 is operated when no baking operation performed, for example prior or after a baking operation. Here, disinfection of the functional subunits can be achieved.

In all it becomes clear that the proposed household appliance allows for disinfection of process media and functional subunits in a comfortable way. Such disinfection can favourably also reduce odours, such that the proposed household appliance can be equipped with a powerful odour reducing functionality.

### List of numerals

- 1: baking oven
- 2: oven muffle
- 3: muffle door
- 4: exhaust ducting
- 5: ventilation opening
- 6: blower
- 7: plasma generator
- 8: plasma generating unit
- 9: aperture
- 10: ventilation unit

## Claims

1. Household appliance (1) adapted for treating objects in a primary treatment process, comprising at least one plasma generator (7, 8) adapted to impinge in a secondary treatment process a disinfecting and/or odour reducing plasma at at least one functional subunit (2, 3, 4, 5) of the appliance.

2. Household appliance (1) according to clam 1, wherein the primary process involves heating foodstuff, in particular cooking or baking, cooling foodstuff, or drying clothing or dishes.

3. Household appliance (1) according to claim 1 or 2, the functional subunit being selected from the group: primary process treatment chamber, in particular oven muffle (2), primary process heating element, primary process cooling element, primary process steam generator, ventilation systems (4, 5, 6), ducting systems (4), in particular exhaust ducting systems (4, 5, 6) and inlet ducting systems, as well as primary process treatment media related storage tanks.

4. Household appliance (1) according to at least one of claims 1 to 3, wherein the plasma generator (7, 8) is adapted to generate a plasma in a primary process treatment medium, preferably of gaseous type, preferably air or steam.

5. Household appliance (1) according to claim 4, further comprising a ventilation unit (10) adapted to ventilate the gaseous type treatment medium through at least one of the at least one plasma generating unit (8).

6. Household appliance () according to at least one of claims 1 to 5, the plasma generator (7, 8) comprising at least one plasma generating unit (8) arranged within or connected to at least one of the at least one functional subunit (2, 3, 4, 5).

7. Household appliance (1) according to at least one of claims 1 to 6, wherein at least one of the at least one plasma generator (7, 8) is integrated into the household appliance (1) or designed as a separate unit releasably connected or connectable to the at least one functional subunit (2, 3, 4, 5).

8. Method of disinfecting at least a functional subunit (2, 3, 4, 5) of a household appliance (1) which is adapted for treating objects in a primary treatment process, wherein in a secondary treatment process a disinfecting plasma generated by at least one plasma generator (7, 8) is impinged at at least one functional subunit (2, 3, 4, 5).

9. Method according to claim 8, wherein the functional subunit is selected from the group: primary process treatment chamber, in particular oven muffle (2), primary process heating element, primary process cooling element, primary process steam generator, ventilation systems (4, 5, 6), ducting systems, in particular exhaust ducting systems (4, 5) and inlet ducting systems, as well as primary process treatment media related storage tanks.

10. Method according to claim 8 or 9, wherein the plasma generator (7, 8) generates a plasma in a primary process treatment medium, preferably of gaseous type, preferably air or steam.

11. Method according to at least one of claims 8 to 10, wherein the gaseous type treatment medium is ventilated through a plasma generating unit (8) of the plasma generator (7, 8) by means of a ventilation unit (10).
